Europäisches Patentamt

European Patent Office

Office européen des brevets

(11) Numéro de publication: **0 126 007**
**A1**

(12) ## DEMANDE DE BREVET EUROPEEN

(21) Numéro de dépôt: **84400976.1**

(22) Date de dépôt: **14.05.84**

(51) Int. Cl.³: **F 16 K 11/20**
G 01 N 33/48, F 16 K 11/085

(30) Priorité: **13.05.83 FR 8307976**

(43) Date de publication de la demande:
**21.11.84 Bulletin 84/47**

(84) Etats contractants désignés:
**AT BE CH DE GB IT LI LU NL SE**

(71) Demandeur: **Ferracci, François**
**113 Boulevard Beaumarchais**
**F-75003 Paris(FR)**

(72) Inventeur: **Ferracci, François**
**113 Boulevard Beaumarchais**
**F-75003 Paris(FR)**

(74) Mandataire: **CABINET BONNET-THIRION**
**95 Boulevard Beaumarchais**
**F-75003 Paris(FR)**

(54) **Dispositif de mesure de caractéristiques d'un liquide, et robinet destiné notamment à un tel dispositif.**

(57) L'invention concerne un dispositif de mesure de caractéristiques d'un liquide, comportant un robinet ayant une clef mobile en rotation dans une cavité prévue dans un bloc formant boisseau, cavité dans laquelle débouchent les orifices de lignes de circulation de fluide.

Selon l'invention, la clef présente une base (61) munie à sa périphérie d'orifices répartis selon un module angulaire déterminé, dont certains sont reliés par des canaux, les orifices de la cavité (60) étant répartis également selon ce module angulaire, et présente également un corps (62) muni d'une chambre (63) où est monté coulissant un piston double (64), un canal (620) reliant l'un des orifices de la base à la chambre, afin que plusieurs lignes puissent être mises en communication entre elles, avec ladite chambre, et séparées, par rotation de la clef.

L'invention est destinée aux laboratoires d'analyse, par exemple pour effectuer des mesures sur des échantillons de sang.

FIG. 2

EP 0 126 007 A1

**0126007**

L'invention concerne un dispositif de mesure des caractéristiques d'un liquide, du type comprenant une pluralité de lignes de circulation de fluide, destiné par exemple aux laboratoires tels que les laboratoires d'analyse, et plus particulièrement les laboratoires d'analyse du domaine médical.

L'invention concerne également un robinet ayant une clef mobile en rotation dans une cavité prévue dans un bloc formant boisseau, cavité dans laquelle débouchent des orifices respectifs appartenant chacun à une ligne de fluide.

En effet, en médecine, le diagnostic et la surveillance de nombreux états pathologiques nécessitent de connaître l'oxygénation et l'état acido-basique du sang du patient.

Cette connaissance peut faire intervenir la mesure ou le calcul de nombreux paramètres : pressions partielles, concentrations, saturations, débits, etc..

Depuis plus de 20 ans, les pressions partielles d'oxygène et de gaz carbonique (dioxyde de carbone) sont mesurées par électrochimie.

La mesure du pH y est habituellement associée, également par méthode électrochimique : potentiométrie directe, à l'aide d'une chaîne de deux électrodes, référence et mesure (méthode de la pile de concentration).

La pression partielle de dioxyde de carbone $P_{CO_2}$ est mesurée par une électrode combinée de pH : la chaîne de mesure est séparée du milieu à analyser par une membrane semi-perméable au dioxyde de carbone gazeux et dissout dans un liquide ; la chaîne de mesure du pH est placée dans un tampon de bicarbonate et l'on y mesure la variation d'activité $H^+$ engendrée dans ce tampon par la variation de concentration de dioxyde de carbone gazeux ayant traversé la membrane.

La pression partielle d'oxygène $P_{O_2}$ est mesurée par voltampérométrie : le courant recueilli est proportionnel à la quantité d'oxygène dissous présent dans le milieu et ce dernier est séparé du capteur par une membrane semi-perméable, sélective pour l'oxygène.

Ces mesures de pressions partielles ne renseignent que sur l'état des fractions dissoutes des gaz. D'autres méthodes et par là même, d'autres instruments sont nécessaires, pour la mesure des concentrations totales des gaz. Les méthodologies sont variées, mais doivent passer par un certain nombre de stades obligatoires :

- pour l'oxygène : libération de la fraction liée chimiquement et retour de la totalité du gaz à une forme dissoute.

- pour le dioxyde de carbone : abaissement du pH du milieu et retour de la fraction ionisée à la forme dissoute.

- dans les deux cas, il existe des méthodes et des appareils de mesure de ces gaz libérés par la mesure de leurs pressions partielles. Parmi les méthodes existantes, on notera plus particulièrement les suivantes :

Concentration totale actuelle en dioxyde de carbone :

L'abaissement brutal du pH du milieu par dilution dans un acide, associé à des phénomènes physiques tels qu'agitation ou vide, entraîne le retour de la forme $HCO_3^-$ (donc ionisée) qui est la forme la plus importante en quantité, vers la forme dioxyde de carbone dissous (gazeux).

On mesure la pression partielle de dioxyde de carbone finale du mélange : la concentration est proportionnelle :

- au coefficient de solubilité de Bunsen,

- à la différence des pressions partielles avant et après mélange de l'échantillon dans l'acide,

- à la dilution, et la concentration $C_{CO_2}$ est :

$$C_{CO_2} = \frac{\alpha}{PB} \times (P_2 - P_1) \times \frac{V}{v} \times 100, \text{ si C est en ml/100 ml,}$$

$\alpha$ : coefficient de Bunsen,

PB : pression barométrique,

$P_1$ : pression partielle en $CO_2$ initiale, avant mélange,

$P_2$ : pression partielle en $CO_2$ finale,

V : volume de réactif acide,

v : volume de l'échantillon.

Concentration totale actuelle en oxygène.

La libération de l'oxygène lié à l'hémoglobine se fait, après hémolyse, par dilution dans une solution de ferricyanure de potassium : on obtient une solution de cyanméthémoglobine. L'oxygène libéré reste dissous.

On mesure la pression partielle de l'oxygène, et la
concentration est proportionnelle :

- au coefficient de solubilité de Bunsen,
- à la différence des pressions partielles,
- à la dilution

et la concentration $C_{O_2}$ est :

$$C_{O_2} = \frac{\alpha}{PB} \times (P_2 - P_1) \times \frac{V}{v} \times 100 \text{ si } C \text{ est exprimé en ml/100ml}$$

Au total : Il existe actuellement :

- des appareils pour la mesure de pH, $P_{CO_2}$ , $P_{O_2}$ ,

- des appareils pour la mesure de $C_{CO_2}$

- des appareils pour la mesure de $C_{O_2}$ .

Par ailleurs, ce sont les mêmes capteurs électrochimiques qui servent à la mesure des pressions partielles et
des concentrations : seul le milieu analysé conditionne la
nature de la mesure :

- sang total "natif" : mesure des pressions partielles,
- sang dilué dans un réactif : mesure des concentrations.

L'invention a pour but de créer un dispositif permettant, à l'aide d'un unique appareil et des mêmes électrodes
de pression partielle d'oxygène et de dioxyde de carbone,
de mesurer successivement, à partir d'un unique échantillon
de sang, les pressions partielles puis les concentrations
d'oxygène et de dioxyde de carbone.

L'invention a également pour but de créer un dispositif
qui soit d'une fabrication pas trop compliquée ni trop onéreuse, tout en restant robuste, fiable et d'une manipulation aisée.

A cet effet, l'invention concerne un dispositif de mesure de caractéristiques d'un liquide, du type comprenant
une pluralité de lignes de circulation de fluide, dispositif
caractérisé en ce qu'il comporte un robinet ayant une clef
mobile en rotation dans une cavité prévue dans un bloc formant boisseau, cavité dans laquelle débouchent des orifices respectifs correspondant chacun à l'une des lignes de fluide, et en ce que ladite clef présente une base de section droite circulaire munie à sa périphérie d'une pluralité d'orifices répartis angulairement selon un module angulaire déterminé et dont certains sont reliés par des canaux percés dans ladite base, les orifices de la cavité étant eux-mêmes répartis angulairement selon le même module angulaire, et présente également un corps muni d'une chambre dans laquelle est monté coulissant un piston double, un canal étant prévu pour relier l'un desdits orifices de ladite base à la dite chambre, de telle sorte que plusieurs lignes puissent être mises en communication entre elles, avec ladite chambre, et séparées par rotation de ladite clef.

L'invention concerne également un robinet ayant une clef mobile en rotation dans une cavité prévue dans un bloc formant boisseau, cavité dans laquelle débouchent des orifices respectifs appartenant chacun à une ligne de fluide, robinet caractérisé en ce que ladite clef présente une base de section droite circulaire munie à sa périphérie d'une pluralité d'orifices répartis angulairement selon un module angulaire déterminé et dont certains sont reliés par des canaux percés dans ladite base, les orifices de la cavité étant eux-mêmes répartis angulairement selon le même module angulaire, et présente également un corps muni d'une chambre dans laquelle est monté coulissant un piston double, un canal étant prévu pour relier l'un desdits orifices de ladite base à ladite chambre, de telle sorte que plusieurs lignes puissent être mises en communication entre elles, avec la dite chambre, et séparées, par rotation de ladite clef.

Les caractéristiques et avantages de l'invention ressortiront d'ailleurs de la description qui va suivre se rapportant à des formes de réalisation préférentielles de l'invention, en référence aux dessins annexés dans lesquels :

la figure 1 représente schématiquement l'ensemble d'un dispositif selon l'invention ;

la figure 2 représente schématiquement, en section longitudinale, un robinet selon l'invention ;

5

la figure 3 représente schématiquement en section transversale selon la ligne III-III de la figure 2, le robinet représenté sur cette figure, dans une première position de sa clef par rapport à son boisseau ;

la figure 4 montre la même section que celle de la figure 3, dans une deuxième position ;

la figure 5 montre la même section que celle des figures 2 et 3, dans une troisième position ;

la figure 6 montre la même section, dans une quatrième position ;

la figure 7 représente schématiquement, en section longitudinale, une deuxième forme de réalisation d'un robinet selon l'invention ;

la figure 8 montre schématiquement une section transversale analogue à celle des figures 3 à 6, d'une autre forme de réalisation.

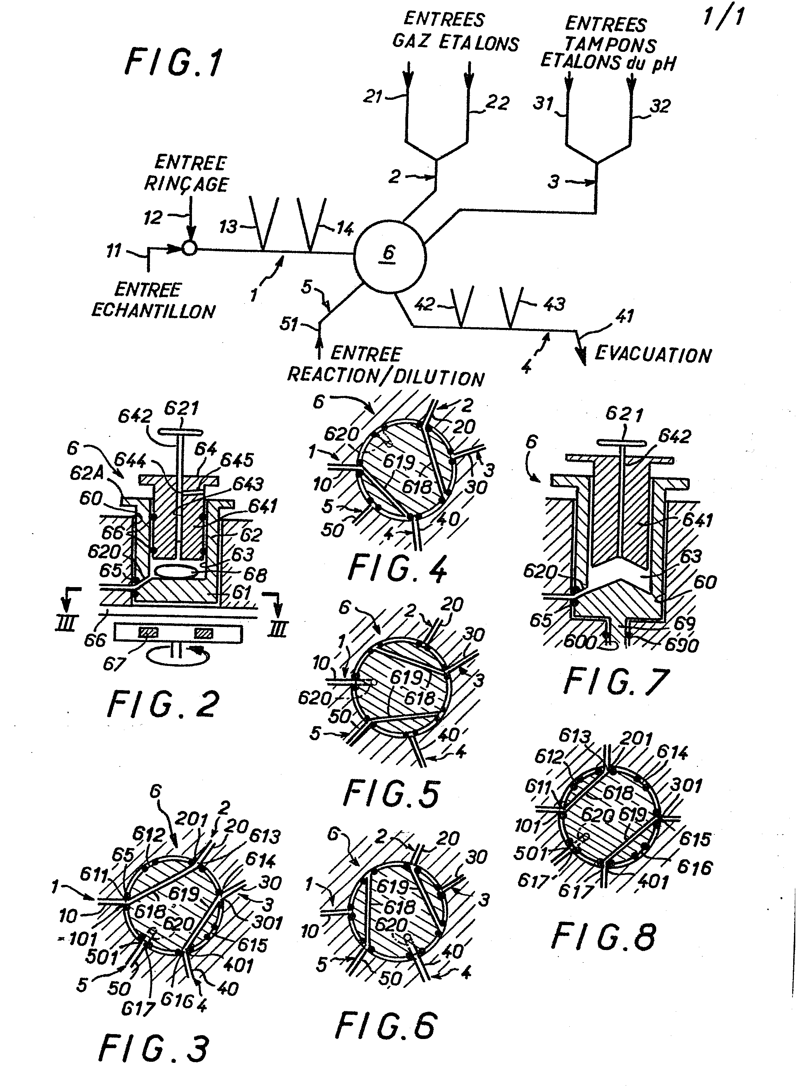

Le dispositif représenté schématiquement sur la figure 1, qui est destiné ici plus particulièrement à mesurer dans un échantillon de sang, les pressions partielles de dioxyde de carbone et d'oxygène en vue du calcul de la concentration de ces gaz dans l'échantillon, ainsi que le pH du sang, comporte, dans une enceinte thermostatée munie de moyens classiques destinés à assurer et à contrôler la circulation de fluides notamment du domaine de la micro-mécanique et de l'électronique, une ligne d'amenée 1 de l'échantillon qui doit faire l'objet des mesures, deux lignes d'amenée et d'étalonnage 2 et 3 correspondant respectivement aux gaz étalons et aux tampons étalons du pH, une ligne de mesure du pH et d'évacuation 4 de l'échantillon, et une ligne d'amenée 5 de réactif destiné à extraire les gaz, ainsi qu'un robinet 6 ayant une clef placée dans une cavité d'un bloc approprié formant boisseau, et destiné à la mise en communication et à la séparation sélectives des différentes lignes lorsque cette mise en communication ou cette séparation est nécessaire. Les sens de circulation de fluide dans ces lignes sont indiqués par des flèches.

La ligne d'amenée 1 de l'échantillon comporte elle-même une entrée 11 pour l'échantillon et une entrée 12 pour un liquide de rinçage, et, entre ces entrées et le robinet 6, une chambre 13 munie d'une sonde ou électrode pour la mesure de la pression partielle de dioxyde de carbone et une chambre 14 munie d'une sonde ou électrode pour la mesure de la pression partielle d'oxygène.

La ligne d'étalonnage 2 comporte deux entrées 21, 22 de gaz étalons, et la ligne d'étalonnage 3 deux entrées 31, 32 de tampons étalons de pH.

La ligne de mesure du pH et d'évacuation 4 comporte, entre le robinet 6 et une sortie 41 vers un évier d'évacuation, une chambre 42 avec une électrode pour la mesure du pH et une chambre avec électrode de référence 43 pour le pH.

La ligne d'amenée 5 de réactif comporte une entrée 51 pour un mélange liquide de réaction/dilution destiné à entraîner les gaz, provenant d'un réservoir (non représenté).

Selon l'invention, le robinet 6 est à voies multiples, et sa clef mobile en rotation comporte un corps creux servant de chambre de réaction/dilution muni d'un double piston; il est associé à un agitateur. Ce robinet est placé au carrefour de toutes les lignes fluidiques énumérées plus haut de telle sorte que celles-ci y aboutissent à des orifices respectifs, afin que la rotation de sa clef provoque la mise en communication ou la séparation de ces lignes.

La clef de ce robinet 6 (figure 2), entièrement de forme générale cylindrique, est ici contenue dans une cavité cylindrique 60 aménagée à cet effet dans le bloc de l'enceinte thermostatée qui a déjà été mentionné, et montée glissante en rotation dans cette cavité 60.

Le bloc formant boisseau est muni (figure 3) de canalisations 10, 20, 30, 40, 50 constituant les parties terminales respectives des lignes de fluide, débouchant dans la cavité 60 par des orifices 101, 201, 301, 401, 501 se succédant dans cet ordre dans le sens des aiguilles d'une montre et distants les uns des autres d'un angle de $\frac{2\pi}{7}$ ou multiple de cet angle ; ainsi les orifices 101 et 201 sont écartés de $2\frac{2\pi}{7}$, les orifices 201 et 301 de $\frac{2\pi}{7}$, les orifices 301 et 401 de $2\frac{2\pi}{7}$, et les orifices 401 et 501, ainsi que 501 et

101, de $\frac{2\pi}{7}$.

La clef du robinet 6 est constituée d'une base pleine 61 et d'un corps creux 62 muni d'une béquille 62A d'actionnement en rotation à sa partie supérieure, dont la partie creuse détermine une chambre 63, la face supérieure de la base 61 constituant le fond de cette chambre 63.

La base 61 comporte, à sa périphérie, sept orifices 611, 612, 613, 614, 615, 616, 617 se succédant également dans cet ordre dans le sens des aiguilles d'une montre, distants l'un par rapport au suivant de $\frac{2\pi}{7}$, de telle sorte que par rotation de la béquille du robinet, l'on puisse amener cinq des orifices de la base vis-à-vis des orifices de la cavité 60.

Un canal capillaire 618 réunit les orifices 611 et 613 distants de $\frac{4\pi}{7}$ et un canal capillaire 619 réunit les orifices 614 et 616 également distants de $\frac{4\pi}{7}$ ; en revanche, les orifices 612 et 615 sont borgnes ou même inexistants, leur positionnement étant alors simplement repéré par la présence de joints toriques d'étanchéité ; un court canal capillaire 620, disposé incliné par rapport à l'horizontale, réunit l'orifice 617, distant de $\frac{2\pi}{7}$ des orifices 611 et 616, et la chambre 63.

Le double piston 64 déjà mentionné est monté coulissant longitudinalement dans la chambre 63 ; ses deux pistons 641, 642 sont montés coaxialement.

Le premier piston 641 est disposé à l'extérieur du second piston 642 et clot la chambre 63 ; son actionnement longitudinal permet de faire varier le volume V de cette chambre 63. Le volume V correspond dans le cas présent à la quantité de réactif nécessaire à la dilution du sang. Sur le plan pratique, ce premier piston 641 est réalisé sous la forme d'une seringue de précision dont le piston intérieur, présentant une course D, constitue ici le second piston 642, qui, en se déplaçant dans une course $\underline{d}$ à l'intérieur d'un canal 643, permet le dosage d'un volume $\underline{v}$ ; dans la zone du canal 643 où l'extrémité libre du piston 642 est susceptible de se déplacer, est prévu un orifice 644 relié par un canal 645 à la périphérie du piston 641, pour la liaison du canal 643 à une installation d'évacuation telle qu'un évier, afin de permettre la purge de la chambre et du capillaire au cours des opérations de remplissage et de nettoyage.

Afin d'assurer l'étanchéité, des joints toriques 65 sont disposés autour des orifices 611, 612, 613, 614, 615, 616, 617, dans des cavités aménagées autour de ces orifices; dans la pratique les orifices borgnes 612 et 615, comme il a été vu, peuvent être tout simplement supprimés, les joints toriques 65 subsistant cependant pour assurer l'étanchéité de la chambre 63 vis-à-vis des canalisations 10, 20, 30, 40 ou 50 en regard ; deux joints toriques 66 assurent l'étanchéité du piston 641 dans la chambre 63, tandis que l'étanchéité du piston 642 est assurée par le fait que ce dernier est recouvert d'un embout en polytétrafluoréthylène.

Des butées (non représentées sur les dessins) limitent la course des pistons 641, 642.

Il a été vu que le robinet est associé à un agitateur ; dans le cas présent, il s'agit d'un agitateur électromagnétique ; ainsi, la face inférieure de la base 61 repose sur un plateau 66 sous lequel est disposé un aimant 67 entraîné par un moteur (non représenté), et, à l'intérieur de la chambre 63, est placé un barreau magnétique 68 dont le mouvement dans la chambre sous l'action du mouvement de l'aimant 67 provoque l'agitation du liquide contenu dans cette chambre.

Les impératifs auxquels doit répondre le robinet :

- faible diffusibilité des gaz dans les matériaux ;

- visibilité des opérations (en particulier pour éliminer le piégeage des bulles de gaz),

et des raisons pratiques, influencent le choix des matériaux constituant le robinet.

Ainsi, la paroi interne du corps creux 62 et le double piston 64 sont en verre, et la base 61 et le revêtement externe du corps creux 62 sont en plexiglas ainsi que la paroi interne de la cavité 60 du bloc thermostaté.

Le thermostatage du bloc est assuré par le fait que celui-ci est plongé dans une enceinte thermostatée (bain-marie ou bloc chauffant) où se trouvent également les autres éléments (cellules de mesure, réservoir de réactif, etc..).

Le fonctionnement du dispositif est le suivant :

On place tout d'abord le robinet 6 dans la position représentée sur la figure 3, que nous désignerons par "position A", dans laquelle la ligne d'amenée 1 de l'échantillon est en communication avec la ligne d'étalonnage 2 grâce au canal 618, la ligne d'étalonnage 3 en communication avec la ligne de mesure du pH et d'évacuation 4 grâce au canal 619, et la ligne d'amenée 5 du réactif en communication avec la chambre 63 et le canal 643 où se déplace le second piston 642, grâce au canal 620. Dans cette position, on étalonne le dispositif de manière classique compte tenu des conditions habituelles de températures et d'humidité, et, dans le même temps, on déplace longitudinalement le piston 641 vers le haut en prélevant le volume V de réactif désiré ; à la fin de cette phase de remplissage, on déplace le piston 641 sur la distance D, ce qui permet la purge du système puis on le repousse à fond de course.

Puis, on place le robinet 6 dans la position représentée sur la figure 4, décalée de $2.\frac{2\pi}{7}$ dans le sens des aiguilles d'une montre par rapport à la position A, que nous désignerons par position B, dans laquelle la ligne d'amenée 1 de l'échantillon est en communication avec la ligne de mesure du pH et d'évacuation 4 grâce au canal 619, les autres lignes étant séparées les unes des autres, c'est-à-dire isolées, par le fait qu'elles sont soit face à un trou borgne, soit face à un canal qui n'est lui-même en communication avec aucune autre ligne. Dans cette position, l'échantillon tel qu'un échantillon de sang est introduit dans la chaîne de mesure et plus particulièrement dans la ligne d'amenée 1, la chambre 13 de mesure de la pression partielle de dioxyde de carbone et la chambre 14 de mesure de la pression partielle d'oxygène, et dans la ligne de mesure du pH et d'évacuation 4 et la chambre 42 pour la mesure du pH.

Ensuite, on place le robinet 6 dans la position représentée sur la figure 5, décalée de $.\frac{2\pi}{7}$ dans le sens inverse des aiguilles d'une montre par rapport à la position B, que nous désignerons par "position C", dans laquelle la ligne d'amenée 1 de l'échantillon est mise en communication avec la chambre 63 et le canal 643 où se déplace le piston 642. Dans cette position, on déplace le piston 642 d'une distance $\underline{d}$ correspondant à une prise d'essai d'un volume $\underline{v}$ de sang, et l'on met l'agitateur en fonctionnement.

Puis on revient en position B, et on effectue les lectures de la pression partielle d'oxygène, de la pression partielle de dioxyde de carbone, et du pH. Après quoi, on procède au rinçage, au nettoyage, et au séchage de la chaîne de mesure, le robinet étant toujours en position B.

Ensuite, on actionne la clef du robinet vers la position C, et on repousse le piston 641 de manière à injecter le mélange sang-réactif dans la ligne d'amenée 1. Puis on lit les nouvelles valeurs des pressions partielles d'oxygène et de dioxyde de carbone, à partir desquelles on effectue les calculs des valeurs des concentrations en oxygène et en dioxyde de carbone par les formules qui ont été données dans le préambule de la demande.

Après cela, on rince, on nettoie et on sèche l'ensemble en passant plusieurs fois de la position C à une position D et réciproquement, en déplaçant alternativement les pistons 641, 642. La position D est définie (figure 6) par la position occupée par le robinet 6 lorsqu'il est décalé de $2.\dfrac{2\pi}{7}$ dans le sens contraire des aiguilles d'une montre par rapport à la position C, c'est-à-dire de $\dfrac{2\pi}{7}$ dans le sens contraire des aiguilles d'une montre par rapport à la position A ; dans cette position D, la chambre 63 est mise en communication avec la ligne de mesure du pH et d'évacuation grâce au canal 620, tandis que les autres lignes sont séparées les unes des autres, c'est-à-dire isolées, par le fait qu'elles sont soit face à un trou borgne, soit face à un canal qui n'est lui-même en communication avec aucune autre ligne.

Enfin, on arrête l'agitateur et soit on fait un nouvel étalonnage, soit on cesse les opérations en mettant l'appareillage au repos.

Comme il a déjà été mentionné, l'exploitation des mesures pour le calcul des concentrations en dioxyde de carbone et en oxygène est effectuée à l'aide des formules qui ont été données dans le préambule de la demande.

Parmi les différents paramètres intervenant dans ces formules, seules les valeurs de $P_2$ sont des variables mesurées.

Il est donc nécessaire de connaître très précisément les autres paramètres :

- $\alpha$ : coefficient de Bunsen relatif à l'eau, à 37° ; cette approximation est justifiée, entraînant une erreur négligeable (0,01 %) ; de même il n'est pas utilisé de valeurs différentes de $\alpha$ pour les mélanges : réactif + eau, et réactif + eau + sang, les facteurs de correction étant négligeables du fait de la faible concentration du mélange réactif, et de la dilution du sang dans ce mélange.

- PB : lue au baromètre mural.

- $P_1$ : pression partielle du mélange réactif avant l'introduction du sang ; pour qu'elle soit connue, on a le choix entre deux possibilités : soit mesurer les pressions partielles d'oxygène et de dioxyde de carbone du réactif avant introduction du sang, mais ceci nécessite de le faire avant chaque détermination, soit tonométrer le mélange réactif avec un gaz titré, et ce, avant son introduction dans la chambre intérieure du robinet. Cette dernière solution paraît préférable si le gaz de tonométrie est judicieusement choisi ; par exemple on peut utiliser de l'azote pur ; dans le montage décrit on utilise préférentiellement l'un des gaz d'étalonnage des électrodes, à savoir le gaz habituellement dénommé "gaz fort" (HI GAZ) ou mélange binaire (10 % $CO_2$, QS $N_2$).

- V : après une première estimation grossière, le volume de la chambre intérieure a été mesuré par gravimétrie à l'eau, la position du piston 641 soigneusement repérée, et les butées installées en conséquence ; la gravimétrie donne ici : V = 1,14829 ml.

- v : la dilution choisie est celle utilisée dans certains appareils de mesure du dioxyde de carbone : 1/50 ème ; connaissant le volume V on mesure le volume v par la serin-

gue de précision que constitue le premier piston 641 en lisant sur des graduations prévues à cet effet le déplacement du second 642, et ici v = 0,0229 ml (22,9 µ l).

Le mélange réactif utilisé a une composition proche de celle du réactif acide mixte décrit par Van Slyke dans sa méthode manométrique de détermination des concentrations en $O_2$ et $CO_2$ ; la modification apportée à cette composition est celle que F. Volter & col. utilise dans la méthode polarographique de détermination du contenu en $O_2$

    - $K_3Fe$ (CN)6 : 0,6 g p. 100 g d'eau distillée,

    - saponine : 0,3 g p. 100 g d'eau distillée,

    - alcool octylique : qs pour saturer la solution,

    - acide lactique : 1 ml pour 100 ml.

Bien entendu, l'invention n'est pas limitée à la forme et au mode de réalisation ci-dessus décrits et représentés, et on pourra prévoir d'autres formes et modes de réalisation sans pour cela sortir du cadre de l'invention.

Ainsi, par exemple, le dispositif d'agitation par barreau magnétique peut être remplacé par un dispositif d'agitation par ultra-sons. Dans ce cas, on peut choisir une fréquence et une puissance qui soient également hémolysantes ; une autre conséquence est alors la suppression de la saponine et de l'alcool octylique du mélange réactif ; de plus, l'emplacement occupé précédemment par le moteur de l'agitateur peut l'être par un moteur relié à la base de la clef du robinet et commandant sa manoeuvre (la sonde génératrice d'ultra-sons étant placée latéralement).

De même, le matériau constituant le piston 641 peut ne pas être transparent : on peut utiliser un métal ou un alliage dont la surface est traitée (oxydation puis téflonnage) pour obtenir une meilleure résistance à la corrosion, une bonne étanchéité de la chambre, et un déplacement plus facile.

Les deux modifications mentionnées ci-dessus permettent également de modifier la forme de la chambre pour obtenir une meilleure circulation des fluides (figure 7).

Dans cette deuxième forme de réalisation, toutes autres choses non mentionnées plus haut et ci-après restant identiques, d'une part, la clef du robinet étant reliée au moteur par un axe 69 passant à l'intérieur d'un puits 600 prévu à la partie inférieure de la cavité 60, l'étanchéité dans le puits 600 est assurée par un joint torique 690, et, d'autre part, le fond de la chambre 63 et la partie inférieure du piston 641 ont une forme générale en cône droit de révolution, le sommet du cône étant disposé en haut.

De même, il est possible de modifier des paramètres de calcul des concentrations tels que ceux portant sur les volumes (par exemple V = 0,5 ml et v = 10 $\mu$ 1) et ceux portant sur la composition du gaz de tonométrie du réactif.

Il est également possible d'apporter des modifications à la disposition des voies du robinet : un autre schéma peut être envisagé soit dans un plan de section, soit dans les trois dimensions ; par exemple (figure 8), on peut prévoir un module égal à $\frac{\pi}{4}$ au lieu de $\frac{2\pi}{7}$; dans ce cas, les orifices 101, 201, 301, 401, 501 sont distants d'un angle de $\frac{\pi}{4}$ ou multiple de cet angle ; plus précisément on peut prévoir que les orifices 101 et 201 soient écartés de $\frac{\pi}{2}$, les orifices 201 et 301 également de $\frac{\pi}{2}$, de même que les orifices 301 et 401, tandis que les orifices 401 et 501, d'une part, et 501 et 101, d'autre part, seraient écartés de $\frac{\pi}{4}$ ; la base 61 comporterait alors huit orifices 611, 612, 613, 614, 615, 616, 617, 617' distants l'un par rapport au suivant de $\frac{\pi}{4}$, un canal 618 réunissant les orifices 611 et 613 distants de $\frac{\pi}{2}$, un canal 619 réunissant les orifices 615 et 617 également distants de $\frac{\pi}{2}$, et un canal 620 réunissant l'orifice 617' équidistant des orifices 611 et 617 et la chambre 63.

On peut aussi envisager de désolidariser la base de la clef, du corps creux contenant la chambre intérieure, ce dernier devenant fixe, seule la base tournant autour de son axe longitudinal ; il faut alors prévoir un joint d'étanchéité entre ces deux parties.

Parmi les autres modifications possibles, on notera pour mémoire qu'un autre acide que l'acide lactique peut être utilisé, la dilution étant choisie selon la nature de cet acide, qu'un joint d'étanchéité circulaire en polytétrafluoréthylène ou un matériau analogue peut être rajouté entre les orifices des voies du robinet, qu'un remplacement des électrodes spécifiques par des capteurs plus sélectifs tels que des transistors à effet de champ est également envisageable.

On peut enfin noter, parmi les nombreux avantages de l'invention, que la disposition des voies du robinet permet à volonté d'effectuer soit la totalité des mesures, soit seulement les mesures classiques de pH et de pressions partielles d'oxygène et de dioxyde de carbone, et également qu'il est possible à la demande de remplacer la clef à double piston par une clef pleine classique, seules les mesures classiques étant alors possibles, ou encore de prévoir le logement d'électrodes de mesure directement dans le robinet et non à distance de celui-ci.

## REVENDICATIONS

1) Dispositif de mesure de caractéristiques d'un liquide, du type comprenant une pluralité de lignes de circulation de fluide, dispositif caractérisé en ce qu'il comporte un robinet (6) ayant une clef mobile en rotation dans une cavité (60) prévue dans un bloc formant boisseau, cavité dans laquelle débouchent des orifices respectifs (101,201, 301,401,501) correspondant chacun à l'une des lignes de fluide (1,2,3,4,5) et en ce que ladite clef présente une base (61) de section droite circulaire munie à sa périphérie d'une pluralité d'orifices (611,612,613,614,615,616,617) répartis angulairement selon un module angulaire déterminé et dont certains (611 et 613,614 et 616) sont reliés par des canaux (618,619) percés dans ladite base, les orifices (101, 201,301,401,501) de la cavité étant eux-mêmes répartis angulairement selon le même module angulaire, et présente également un corps (62) muni d'une chambre (63) dans laquelle est monté coulissant un piston double (64), un canal (620) étant prévu pour relier l'un desdits orifices (617) de la dite base (61) à la chambre (63), de telle sorte que plusieurs lignes (1,2,3,4,5) puissent être mises en communication entre elles, avec ladite chambre (63), et séparées, par rotation de ladite clef.

2) Dispositif du type robinet ayant une clef mobile en rotation dans une cavité prévue dans un bloc formant boisseau, cavité dans laquelle débouchent des orifices respectifs appartenant chacun à une ligne de fluide, robinet caractérisé en ce que ladite clef présente une base (61) de section droite circulaire munie à sa périphérie d'une pluralité d'orifices (611,612,613,614,615,616,617) répartis angulairement selon un module angulaire déterminé et dont certains (611 et 613, 614 et 616) sont reliés par des canaux (618,619) percés dans ladite base, les orifices (101,201, 301,401,501) de la cavité (60) étant eux-mêmes répartis angulairement selon le même module angulaire, et présente également un corps (62) muni d'une chambre (63) dans laquelle est monté coulissant un piston double (64), un canal (620) étant prévu pour relier l'un desdits orifices (617) de ladite base (61) à ladite chambre (63), de telle sorte que plusieurs lignes (1,2,3,4,5) puissent être mises en communication entre elles, avec ladite chambre (63), et séparées, par rotation de ladite clef.

3) Dispositif selon l'une quelconque des revendications 1 et 2, caractérisé en ce que ladite base (61) est munie d'orifices (611,612,613,614,615,616,617) espacés angulairement selon un module angulaire déterminé, deux orifices (611 et 613) espacés du double du module étant réunis par un premier canal (618), deux autres orifices (614,616) espacés également du double du module étant réunis par un deuxième canal (619), un orifice (617) équidistant d'un orifice (611) du premier canal (618) et d'un orifice (616) du deuxième canal (619) étant réuni à ladite chambre (63) par un troisième canal (620).

4) Dispositif selon l'une quelconque des revendications 1 à 3, caractérisé en ce que le module est égal à $\frac{2\pi}{7}$.

5) Dispositif selon l'une quelconque des revendications 1 à 3, caractérisé en ce que le module est égal à $\frac{\pi}{4}$.

6) Dispositif selon l'une quelconque des revendications 1 à 5, caractérisé en ce que le piston double (64) comporte deux pistons (641,642) montés coaxialement, le piston extérieur (641) déterminant une paroi mobile de ladite chambre (63), destinée à clore cette chambre.

7) Dispositif selon l'une quelconque des revendications 1 à 6, caractérisé en ce que le piston double (64) comporte deux pistons (641,642) montés coaxialement, le piston intérieur (642) étant monté mobile dans un canal (643) comportant un orifice (644) relié lui-même par un canal (645) à la périphérie du piston extérieur (641).

8) Dispositif selon l'une quelconque des revendications 1 à 7, caractérisé en ce que la chambre (63) et le piston double (64) comportent respectivement un fond et une partie inférieure coniques.

9) Dispositif selon l'une quelconque des revendications 1 à 8, caractérisé en ce qu'il comporte des moyens d'agitation (67,68) agissant sur le liquide contenu dans la chambre (63).

**0126007**

10) Dispositif selon la revendication 1, caractérisé en ce qu'il comporte cinq lignes (1,2,3,4,5) aboutissant à un dispositif du type robinet, à savoir une ligne d'amenée (1) comportant une entrée (11) pour un échantillon de liquide sur lequel des mesures doivent être effectuées et une entrée (12) pour un liquide de rinçage, ainsi que des chambres (13,14) de mesure de pressions partielles, une ligne d'étalonnage (2) comportant des entrées (21,22) de gaz étalons, une ligne d'étalonnage (3) comportant deux entrées (31,32) de tampons étalons, une ligne de mesure et d'évacuation (4) comportant des chambres (42,43) de mesure et de référence, et une ligne d'amenée de réactif (15), chacune desdites lignes (1,2,3,4,5) aboutissant au boisseau du robinet (6) et plus particulièrement à un orifice (101,201, 301,401,501) dudit boisseau, les orifices (101,201,301,401, 501) du boisseau étant répartis angulairement selon ledit module angulaire déterminé.

FIG.1 — FIG.8

0126007

8447

0126007
Numéro de la demande

## Office européen des brevets

# RAPPORT DE RECHERCHE EUROPEENNE

EP 84 40 0976

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | FR-A-2 276 585 (INSTRUMENTATION LABORATORY INC.) * Revendications, point 1; figures * | 1 | F 16 K 11/20<br>G 01 N 33/48<br>F 16 K 11/085 |
| A | FR-A-1 394 938 (S.E.D.A.) | | |

DOMAINES TECHNIQUES RECHERCHES (Int. Cl. ³)

F 16 K
G 01 N

Le présent rapport de recherche a été établi pour toutes les revendications

| Lieu de la recherche | Date d'achèvement de la recherche | Examinateur |
|---|---|---|
| LA HAYE | 16-08-1984 | VAN REETH A.L.J. |